# EUROPEAN PATENT SPECIFICATION

(11) **EP 2 101 852 B1**
(45) Date of publication and mention of the grant of the patent: **08.07.2015**
(21) Application number: 07856845.8
(22) Date of filing: 18.12.2007
(51) Int. Cl.: A61M 16/04

(54) **ENDOTRACHEAL DEVICE FOR MECHANICAL VENTILATION**
ENDOTRACHEALVORRICHTUNG ZUR MECHANISCHEN BEATMUNG
DISPOSITIF ENDOTRACHÉAL POUR VENTILATION MÉCANIQUE

(30) Priority: 21.12.2006 IT MI20062476
(43) Date of publication of application: 23.09.2009
(73) Proprietor: Tagliavini, Leonardo, 43038 Fraz. Castellaro (IT)
(72) Inventor: Tagliavini, Leonardo, 43038 Fraz. Castellaro (IT)
(74) Representative: Lunati, Valerio
(86) International application number: PCT/EP2007/011118
(87) International publication number: WO 2008/074468

(56) References cited:
- DE-A1- 4 009 468
- DE-A1- 19 506 212
- US-A- 4 423 725
- US-A1- 2001 054 425
- US-B1- 6 242 472

## Description

The present invention relates to an endotracheal device for mechanical ventilation, of the type indicated in the preamble of claim 1. Similar devices are described in patent documents: DE-A-19506212, US-A-6242472, US-A-4423725, DE-A-4009468.

Endotracheal devices for mechanical ventilation are also known as endotracheal tubes, used in medicine on patients during anaesthesia, in emergencies and intensive care.

They are suitable to provide mechanical ventilation to the patient in conditions in which he or she cannot breathe normally.

These tubes comprise two ports, one distal and one proximal, positioned substantially at the ends of this tube and in communication with each other through the inside of the tube.

They are positioned so that one part, comprising the distal port, is inserted into the trachea of the patient, while the other part, comprising the proximal port, is located outside the trachea and the oral or nasal cavity of the patient.

The proximal port is then connected to specific machines for mechanical ventilation, which deliver air to and expel air from the human body through the endotracheal tube, so as to implement mechanical ventilation. Endotracheal tubes must therefore be able to be inserted into and removed from the trachea of patients and moreover must guarantee at least a partial pneumatic seal inside the trachea, necessary to carry out mechanical ventilation.

For this purpose, they have a diameter smaller than the diameter of the trachea, so that they can be inserted therein and removed therefrom, and comprise, if intended for intubation of adult patients, a specific inflatable cuff, placed in proximity of the distal port and suitable to provide pneumatic seal of the tube inside the trachea. In fact, when the cuff is deflated it adheres to the outer wall of the tube and allows insertion and removal of the tube, while when it is inflated it adheres to the inner tracheal wall, so as to allow pneumatic seal of the tube inside the trachea.

Differently, endotracheal tubes intended for patients in paediatric age are not provided with cuffs.

Tracheal tubes therefore comprise ducts, called lumens, positioned inside the wall of this tube and suitable to place said cuff in communication with an outer valve.

The fluid to inflate the cuff is made to pass through said ducts or lumens. The outer valve is located outside the oral cavity and allows the cuff to be inflated by means of a needle-free syringe or the like.

It is thus possible and easy to inflate the cuff to specific pressures and deflate the cuff to allow pneumatic seal, or removal and insertion, of the tube.

The aforesaid prior art has some important drawbacks.

In fact, endotracheal tubes tend to become the site of bacterial proliferation and accumulation of endogenous secretions only a few hours after intubation, at the level of the contact area between the endotracheal tube and the trachea.

Patients that have been intubated for several hours may therefore be subject to proliferation of pathogenic agents and formation of a sort of film known as biofilm, favouring proliferation and colonization of the tube.

All this provides for an increase in the incidence of infectious diseases of the pulmonary system, caused by accumulation of high bacterial loads at the level of the upper airways and passing into the lower airways.

Attempts have been made to remedy the aforesaid drawback through different solutions.

A first solution was to place an antibacterial agent, realized, for example, by chlorhexidine, silver salts or antibiotics, on the cuff.

However, this solution seems to only partly solve the problem.

A different widely used solution provides for the implementation of a lumen or suction duct inside the tube.

This suction lumen has a distal port at the level of the top of the cuff and in particular placed on the outer wall of the tube, and a proximal port outside the oral cavity of the patient, connectable to a ventilation machine or the like.

Therefore, it is possible to aspirate body secretions produced above the cuff through the suction lumen and prevent, at least partly, the creation of said biofilm.

The suction lumen has been improved by adding, in proximity of the distal port thereof, special elements, realized by small cuffs not in contact with the tracheal wall or thicknesses projecting from the outer wall of said tube. These elements make it possible to prevent the distal port of the suction lumen, placed at the level of the outer wall of the tube, from coming into contact with the tracheal wall, causing clogging of this port and consequently making suction impossible.

Notwithstanding continuous improvements, the suction lumen does not completely solve the aforesaid drawbacks.

In particular, areas in which pathogenic agents accumulate, which are difficult to reach by suction, can form in the interstices between the cuff and the tracheal wall.

Moreover, ulceration can form in the trachea of the patient in the contact area with the endotracheal tube and with the various thicknesses and devices connected thereto.

Therefore, the problem of accumulation of pathogenic agents and the like in proximity of the cuff remains.

In this situation, the technical aim of the present invention is to invent an endotracheal device for mechanical ventilation that is able to substantially overcome the aforesaid drawbacks.

Within said technical aim, an important object of the invention is to realize an endotracheal device for mechanical ventilation capable of preventing accumulation and proliferation of pathogenic agents in proximity of said cuff also after intubation of patients for periods of several days.

The technical aim and the objects specified are achieved by an endotracheal device for mechanical ventilation characterized in that it comprises one or more of the new technical solutions described and claimed below.

The accompanying drawings show by way of example preferred embodiments of the invention. In particular:

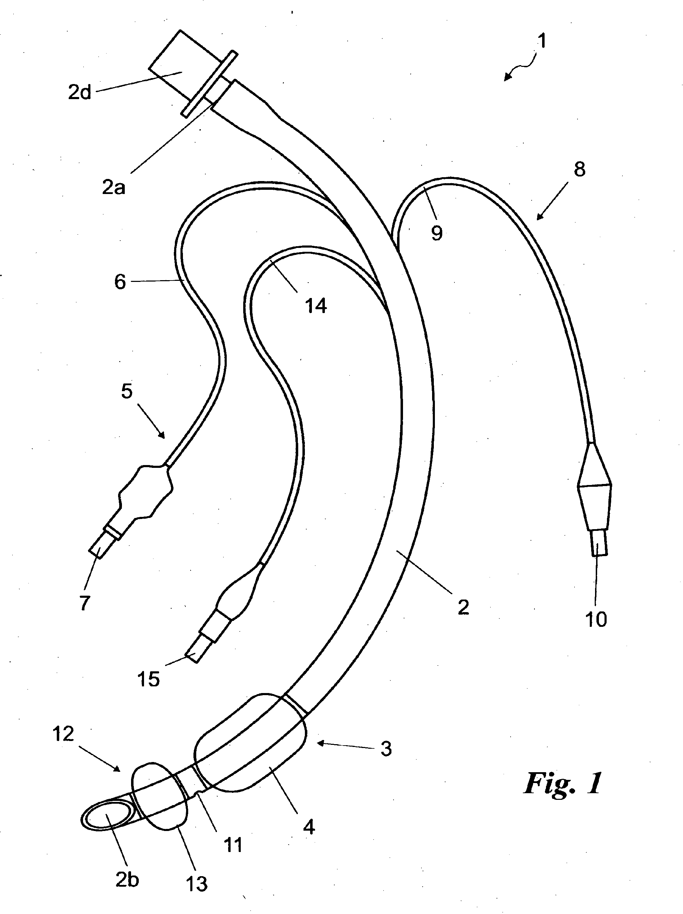

**Fig. 1** show a device according to the invention;
**Fig. 2** shows the normal section, along the line II-II, of the device according to the invention;
**Fig. 3** shows a longitudinal section, along the line III-III, of the device according to the invention;
**Fig. 4a** shows a first step of use of the device according to the invention;
**Fig. 4b** shows a second step of use of the device according to the invention; and
**Fig. 4c** shows a third step of use of the device according to the invention.

With reference to the Figures, the endotracheal device for mechanical ventilation according to the invention is indicated as a whole with the number **1**.

It is suitable to be partly positioned inside the trachea T of a patient, and to allow fluid connection between the respiratory tract of the patient and a machine for mechanical ventilation, of known type and not described herein. The device 1 comprises a ventilation tube **2.**

This tube 2 realizes the communication channel between the ventilator machine and the respiratory tract of the patient. It is appropriately made of specific and known polymer materials, has a diameter of between 0.5 and 2 cm, and a variable length in the order of twenty or thirty centimetres.

It also comprises a proximal port **2a,** suitable to be constrained to a machine for mechanical ventilation, and a distal port **2b** in fluid communication with the proximal port 2a.

In particular, the proximal port 2a comprises a coupling **2d,** of standard shape and dimensions and conforming with the characteristics of the machine for mechanical ventilation.

The distal port 2b is instead preferably bevelled.

The device **1** also comprises primary seal means **3,** suitable to allow sealing of the device 1 inside the trachea.

In particular, the primary seal means 3 of the device 1 comprise an inflatable and deflatable primary cuff **4** suitable to allow sealing or insertion of the device 1 inside the trachea.

This cuff 4 is of substantially known type and is inflatable and deflatable through specific expansion and contraction means **5.**

More in particular, the primary cuff 4 is substantially realized by a flexible sheath made of polymer material, which when deflated adheres substantially to the outer face of the wall **2c** of the tube 2, while when inflated it adheres substantially to the tracheal wall, guaranteeing pneumatic seal of this tube 2 inside the trachea.

It is placed at a distance of preferably between 2 cm and 5 cm from the distal port 2a and has a length of preferably between 2 cm and 6 cm.

The means 5 are also of known type and comprise a first inflation channel or lumen **6** of the primary cuff **4** and an access valve **7** to said first inflation lumen 6.

In particular, the inflation lumen 6 is realized by a flexible tube with a diameter of between 0.3 and 3 mm, and a length preferably exceeding the length of the tube 2.

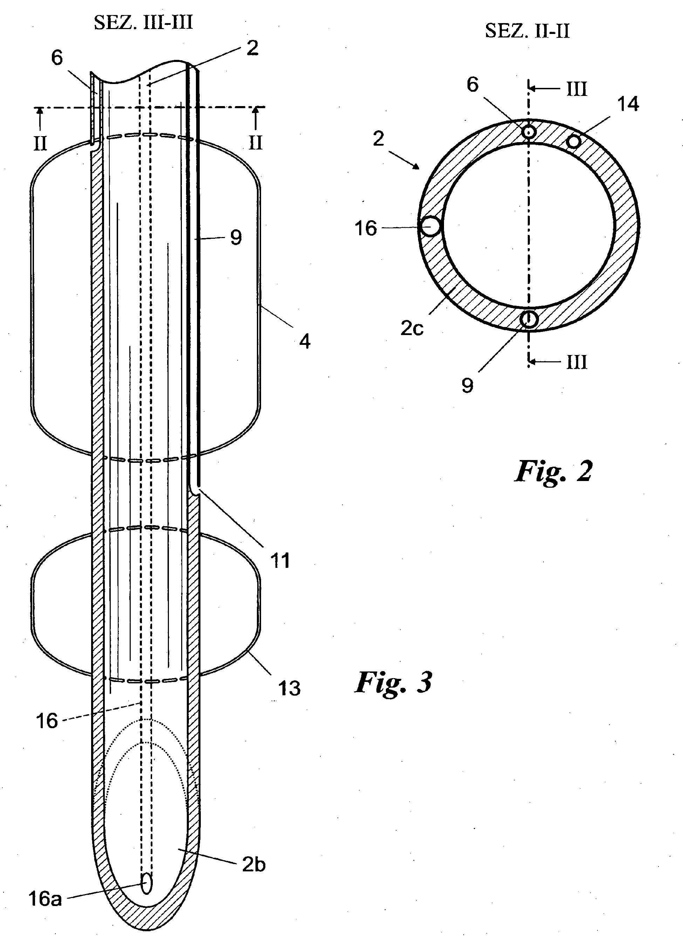

This lumen 6 extends partly inside the wall 2c of the tube 2, as shown in the section of tube 2 in Fig. 2, and partly outside this tube.

In particular, the portion positioned inside the wall 2b is in fluid communication with the primary cuff 4 and the portion on the outside of the wall 2b is in fluid communication with the access valve 7.

This valve 7 allows the pressure inside the primary cuff 4 to be adjusted and maintained constant. In particular, the valve 7 is configured so that the cuff 4 can be accessed by means of a needle-free syringe, as mentioned in the prior art description, or by means of similar known means.

The device 1 also comprises irrigation means **8** of the trachea suitable to allow irrigation of the trachea in proximity of the primary cuff **4.**

In particular, the irrigation means 8 comprise an irrigation channel or lumen **9.**

The irrigation lumen 8 is realized by a flexible tube with a diameter of between 0.3 mm and 3 mm, and a length preferably exceeding the length of the tube 2, and extends partly inside the wall 2c of the tube 2, as shown in the section of this tube 2 in fig. 2, and partly outside this tube.

The portion of the lumen 8 outside the tube 2 preferably terminates with a valve **10** or with a simple port suitable to allow an irrigation fluid, and in particular a liquid and more in particular a physiological solution, to flow. Also in this case the valve 10 is appropriately suitable to be opened by means of a needle-free syringe.

The portion inside the wall 2c of the lumen 8 instead terminates with one or more ports **11,** appropriately positioned in proximity of the primary cuff 4 and produced on the outer face of the wall 2c.

In particular, a single port 11 can be positioned in distal position with respect to the primary cuff 4, as shown in Fig. 3, or alternatively in proximal position with respect to said cuff 4.

Differently, two ports 11 can be positioned in distal and proximal positions or more than two ports 11 positioned in opposite, or in any case different, angular positions with respect to the centre of the tube 2.

Moreover, these ports can be accompanied by a plurality of irrigation lumens 8.

In particular, three ports 11 can be provided, positioned symmetrically along a single circumferential sector of the tube 2.

Moreover, as shown in Fig. 3, the port 11 preferably has direction inclined with respect to the normal direction of the axis of the tube 2. This inclined direction is such as to direct the flow of irrigation fluid delivered from the port 11 towards the area of the primary cuff 4 and to favour irrigation of the trachea at the level and in proximity of said primary cuff 4.

The device 1 also appropriately comprises secondary seal means **12** of the device 1 inside the trachea, suitable to allow at least temporary pneumatic seal of the device 1 inside the trachea.

In particular, said secondary seal means 12 comprise a secondary cuff **13** placed in proximity of the primary cuff 4.

Said secondary cuff 13 is preferably placed in distal position with respect to the primary cuff 4 and at a distance of between 0.5 mm and 20 mm therefrom.

It is appropriately realized in a manner very similar to the primary cuff 4 but is appropriately shorter in length, in the direction of the axis of the tube 2, with respect to the primary cuff 4. In particular, the secondary cuff 13 has a length of appropriately between 0.5 cm and 3 cm. Nonetheless, the secondary cuff 13 could have a length equal to the length of the primary cuff.

Where the secondary cuff 13 is present, the port 11 of the irrigation means is appropriately placed between said primary cuff 4 and said secondary cuff 13, as shown in Fig. 3.

Moreover, the secondary seal means 12 comprise a second inflation lumen **14** of the secondary cuff 13 and a second access valve 15 to said second inflation lumen 14.

The second inflation lumen 14 and the second access valve 15, which allow inflation and deflation of the secondary cuff 13, are similar to the first inflation lumen 6 and to the access valve 7 which allow inflation and deflation of the primary cuff 4 and are separate therefrom.

Also in this case the valve 15 is appropriately configured so that the cuff 13 can be accessed by means of a needle-free syringe, or by means of similar devices.

The device 1 can also comprise a secondary lumen **16,** suitable to allow irrigation of the distal area of the trachea with respect to the secondary cuff 13.

Said distal lumen 16 comprises a distal port **16a** placed at the level of the longer portion of the distal port 2b, of the tube 2, and in distal position with respect to the secondary cuff 13.

The distal lumen 16 can also be suitable to allow the suction of any liquids, i.e. irrigation liquid or any tracheobronchial secretions, or the inoculation of substances directly into the respiratory system of the patient.

The device 1 can also comprise a suction lumen, not shown in the accompanying figures.

This suction lumen is realized according to the previously described prior art and is similar to the lumens described, and in particular preferably has one or more ports in proximity of the primary cuff 4 which can be placed both in distal position and in proximal position with respect to said cuff 4. In particular, it can be placed between the secondary and the primary cuff. Finally, a safety system can be provided to close the ports 11 in the case in which the primary cuff 4 is inflated.

This system can be based on the presence of a valve placed in proximity of said ports which closes due to the increased pressure present inside the primary cuff 4, or otherwise by a dual valve which exclusively allows simultaneous opening and closing of the access valve 10 of the irrigation lumen 9 and of the access valve 7 of the first inflation lumen 6.

Operation of the endotracheal device 1, the structure of which is described above, is as follows.

The device 1 is positioned with the two cuffs, primary 4 and secondary 13, deflated, so that it can be inserted into the trachea of a patient.

After being inserted into the trachea, the primary cuff 4 is inflated through the specific means 5.

In particular, the primary cuff 4 is inflated to a pressure variable as a function of the patient and on average of between 14 cm_{H2O} and 20 cm_{H2O} (centimetres of water, 1 cm_{H2O} is equal to 98.0638 Pascal).

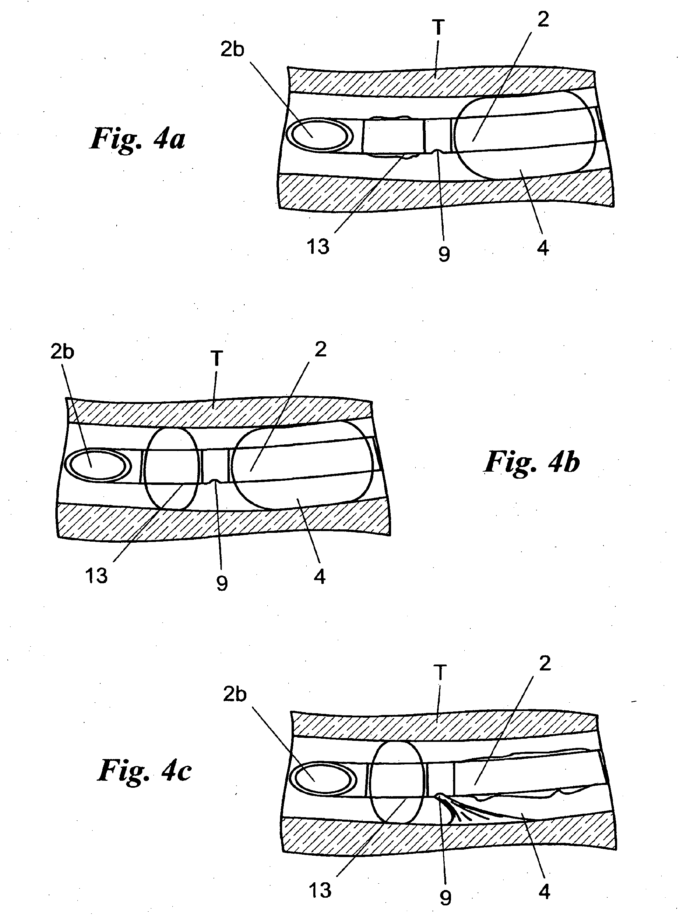

The device 1 then operates as an endotracheal tube (Fig. 4a) described in prior art. Moreover, if it comprises one or more suction lumens, it can continuously aspirate secretions produced by the human body above the primary cuff 4.

After a period of ventilation, normally of between 6 and 8 hours, a process to irrigate the trachea is carried out.

This process is shown in Figs. 4a, 4b and 4c.

In a first step (Fig. 4b) of said process, the secondary cuff 13 is inflated, through the lumen 14 and the valve 15. In particular, when the length the secondary cuff 13 is less than the width of the primary cuff 4, it is inflated to higher pressures with respect to the inflation pressures of the primary cuff 4, so as to guarantee the pneumatic seal of the device 1 notwithstanding a decreased width. These pressures are variable as a function of the patient and on average are between 18 cm_{H2O} and 25 cm_{H2O}. In a second step (Fig. 4c) only the primary cuff 4 is deflated, so that the pneumatic seal of the device 1 is guaranteed by the secondary cuff 13, and the trachea is irrigated through the described irrigation means 8.

In particular, an irrigation liquid is injected through the valve 10 using a needle-free syringe.

The irrigation liquid then flows inside the irrigation lumen 9 and is delivered through the port 11 or ports 11.

The delivered liquid then flows in particular along the tracheal wall at the level and in proximity of the area previously involved by the presence of the primary cuff 4. The liquid also appropriately flows over the device 1 and in particular at the level of the primary cuff 4.

During this operation the liquid eliminates any accumulations of bacteria, or biofilms, that have formed in the area due to the presence of the cuff 4. This liquid is fed with a pressure determined by the action of the syringe plunger. This pressure is variable according to the dimension of the tube 2 and of the trachea of the patient, as well as other variables, and is therefore appropriately manually regulated.

The liquid subsequently flows through the trachea and reaches the oral cavity, where it is appropriately aspirated with known means not belonging to the device 1.

Alternatively, the liquid can be aspirated by the suction lumen or directly by the irrigation lumen 9.

This irritation liquid is preferably composed of a physiological solution to which other irrigation liquids can be added.

After this operation, the duration of which is only a few minutes, the classic configuration of the device 1 is restored, however always maintaining the pneumatic seal between the device 1 and the trachea.

In particular, the primary cuff 4 is inflated and, subsequently, the secondary cuff 13 is deflated.

After the irrigation process has been terminated, another period of ventilation is allowed to pass before repeating the irrigation process. Moreover, the device 1, and in particular the distal lumen 16, allows suction of any irrigation liquid that could be present in the area between the endotracheal tube and the trachea and irrigation of the secondary cuff 13 and of the distal portion of the endotracheal tube 2.

In particular, to carry out suction of the irrigation liquid present in the area between the endotracheal tube and the trachea, it is necessary to: maintain both cuffs 4 and 13 deflated; if necessary occlude, for a few seconds, the endotracheal tube 2; aspirate said irrigation liquid from the distal lumen 16. Differently, to irrigate the secondary cuff 13 and the distal portion of the endotracheal tube 2 it is necessary to: if necessary inflate the primary cuff 4; if necessary occlude, for a few seconds, the endotracheal tube 2; irrigate the secondary cuff 13 through the irrigation lumen 9 and aspirate said irrigation liquid from the distal lumen 16.

The invention achieves important advantages.

In fact, the device 1, through the irrigation means 8, prevents pathogenic agents from accumulating and proliferating in proximity of the cuff 4. Moreover, the device 1 combats proliferation and accumulation of pathogenic agents not only at the level of the cuff 4, but also in the area proximal thereto and the oral cavity. Therefore, action of the device 1 is also carried out at retrolaryngeal and oral level, often the site of passage and active proliferation of pathogenic agents.

The device 1 can also be maintained inside the oral.cavity for any period of time; in fact, by irrigating the trachea as described at appropriate intervals, accumulation of pathogenic agents and formations of bacterial biofilms can be avoided.

A further advantage is given by the fact that the device 1 moreover does not cause ulceration of the trachea or of other structures, which can form due to forced suction and pressure.

Another advantage is due to the fact that the device 1 does not use permanent antibacterial agents and antibiotics.

Last but not least advantage is given by the possible suction of tracheobronchial secretions and by the possibility of direct inoculation of any substances into the lower airways through the distal lumen 16.

The invention is susceptible to modifications and variants falling within the aim of the inventive concept.

In particular, if the device 1 is used for patients in paediatric age it is not provided with the primary cuff 4, but comprises the irrigation means 8 and appropriately the secondary seal means 12.

A similar device may be used also for tracheostomic cannulas, which have the same problems of endotracheal devices.

All parts are replaceable by equivalent elements and the materials, forms and dimensions can be any.

## Claims

1. Endotracheal device for mechanical ventilation suitable to be partly positioned inside the trachea (T) of a patient, and comprising:
- a ventilation tube (2) including at least a proximal port (2a), suitable to be constrained to a machine for mechanical ventilation, and at least a distal port (2b) in fluid communication with said proximal port (2a),
- primary seal means (3) suitable to allow sealing of said device (1) inside the trachea and comprising an inflatable and deflatable primary cuff (4),
- irrigation means (8) of said trachea suitable to allow irrigation operations in proximity of said primary cuff (4),
- secondary seal means (12) of said device (1) in said trachea, suitable to allow at least temporary sealing of said device (1) inside said trachea and comprising a secondary cuff (13) placed in proximity of said primary seal means (3),
- wherein said irrigation means (8) comprise at least an irrigation lumen (9), suitable to allow the flow of an irrigation fluid from the outside to the inside of said trachea,
- wherein said irrigation lumen (9) terminates with at least one or more ports (11) having a direction inclined with respect to the normal direction of the axis of the tube (2) such as to direct the flow of irrigation fluid delivered from the port (11) towards the area of said primary cuff (4) and to favour irrigation of the trachea at the level and in proximity of said primary cuff (4),
**characterized in that** said secondary cuff (13) is placed in distal position with respect to said primary cuff (4).

2. Device according to one Claim 1, wherein said secondary cuff (13) has a length in axial direction of said tube (2) shorter with respect to the length of said primary cuff (4).

3. Device according to one or more of the preceding claims, in particular Claim 1, wherein said secondary seal means (12) comprise a second inflation lumen (14), suitable to inflate and deflate said secondary cuff (13) separately from said primary cuff (4).

4. Device according to one or more of the preceding claims, in particular Claim 1, wherein said irrigation lumen (9) includes at least a port (11) placed in proximity of said primary cuff (4).

5. Device according to one or more of the preceding claims, in particular Claim 4, wherein said port (11) is placed in distal position with respect to said primary cuff (4).

6. Device according to one or more of the preceding claims, in particular Claim 1, wherein said irrigation lumen (9) is partly positioned inside the wall (2b) of said ventilation tube (2).

7. Device according to one or more of the preceding claims, in particular Claim 1, comprising a distal lumen (16) suitable to allow inoculation of substances directly into the respiratory system of the patient.

## Patentansprüche

1. Endotrachealvorrichtung zur mechanischen Beatmung, die geeignet ist, teilweise in das Innere der Luftröhre (T) eines Patienten eingeführt zu werden und Folgendes umfasst:
- einen Beatmungstubus (2) mit wenigstens einer proximalen Öffnung (2a), die geeignet ist, an eine Maschine zur künstlichen Beatmung angeschlossen zu werden, und wenigstens einer mit der genannten proximalen Öffnung (2a) für den Mediumdurchfluss verbundenen distalen Öffnung (2a),
- Hauptabdichtvorrichtungen (3), die geeignet sind, die Dichtheit der genannten Vorrichtung (1) im Inneren der Luftröhre zu gewährleisten und einen aufblasbaren und entleerbaren Hauptcuff (4) umfassen,
- Spülvorrichtungen (8) zum Spülen der genannten Luftröhre, die geeignet sind, Spülvorgänge in der Nähe des Hauptcuffs (4) zu gestatten.
- Hilfsabdichtvorrichtungen (12) der genannten Vorrichtung (1) in der genannten Luftröhre, die geeignet sind, wenigstens vorübergehend die Dichtheit der genannten Vorrichtung (1) im Inneren der genannten Luftröhre zu gewährleisten,
- bei der die genannten Spülvorrichtungen (8) wenigstens ein Spüllumen (9) umfassen, das geeignet ist, das Durchfließen eines Spülmediums von außen in das Innere der genannten Luftröhre zu gestatten,
- bei der das genannte Spüllumen (9) dagegen mit einer oder zwei Öffnungen (11) mit einem geneigten Verlauf im Verhältnis zur normalen Richtung der Achse des Tubus (2) endet, so dass der aus der Öffnung (11) kommende Spülmediumfluss zu dem Bereich des genannten Hauptcuffs (4) geleitet und das Spülen der Luftröhre auf dem und in der Nähe des Hauptcuffs (4) begünstigt wird,
**dadurch gekennzeichnet, dass**:
- der genannte Nebencuff (13) sich im Verhältnis zum genannten Hauptcuff (4) in distaler Position befindet.

2. Vorrichtung nach einem oder mehreren der vorangegangenen Ansprüche, insbesondere des Anspruchs 1, bei der der genannte Nebencuff (13) in axialer Richtung des genannten Tubus (2) eine geringere Länge als die des genannten Hauptcuffs (4) aufweist.

3. Vorrichtung nach einem oder mehreren der vorangegangenen Ansprüche, insbesondere Anspruch 1, bei der die genannten Hilfsabdichtvorrichtungen (12) ein zweites Aufblaslumen (14) umfassen, das geeignet ist, den genannten Nebencuff (13) unabhängig von dem genannten Hauptcuff (4) aufzublasen und zu entleeren.

4. Vorrichtung nach einem oder mehreren der vorangegangenen Ansprüche, insbesondere Anspruch 1, bei der das genannte Spüllumen (9) wenigstens eine Öffnung (11) in der Nähe des genannten Hauptcuffs (4) umfasst.

5. Vorrichtung nach einem oder mehreren der vorangegangenen Ansprüche, insbesondere Anspruch 4, bei der die genannte Öffnung (11) im Verhältnis zu dem genannten Hauptcuff (4) in distaler Position angeordnet ist.

6. Vorrichtung nach einem oder mehreren der vorangegangenen Ansprüche, insbesondere Anspruch 1, bei der das genannte Spüllumen (9) teilweise im Inneren der Wand (2b) des genannten Beatmungstubus (2) angeordnet ist.

7. Vorrichtung nach einem oder mehreren der vorangegangenen Ansprüche, insbesondere Anspruch 1, die ein distales Lumen (16) umfasst, das geeignet ist, das Injizieren von Substanzen direkt in das Innere des Atmungssystems des Patienten zu gestatten.

## Revendications

1. Dispositif endotrachéal pour ventilation mécanique conçu pour être partiellement placé à l'intérieur de la trachée (T) d'un patient, et comprenant :
- un tube de ventilation (2) comprenant au moins un orifice proximal (2a), conçu pour être fixé à une machine pour la respiration artificielle, et au moins un orifice distal (2b) en communication fluidique avec ledit orifice proximal (2a),
- moyens de retenue principaux (3), conçus pour permettre la retenue dudit dispositif (1) à l'intérieur de la trachée, et comprenant un ballonnet primaire (4) gonflable et dégonflable,
- moyens de lavage (8) de ladite trachée conçus pour permettre le lavage à proximité dudit ballonnet principal (4).
- moyens de retenue auxiliaires (12) dudit dispositif (1) à l'intérieur de ladite trachée, conçus pour permettre au moins temporairement la retenue dudit dispositif (1) à l'intérieur de ladite trachée,
- dans lequel lesdits moyens de lavage (8) comprennent au moins un lumen de lavage (9), conçu pour permettre l'écoulement d'un fluide de lavage de l'extérieur à l'intérieur de la trachée,
- dans lequel ledit lumen de lavage (9) se termine par contre avec un ou plusieurs orifices (11) ayant une direction inclinée par rapport à la direction normale à l'axe du tube (2) telle à diriger le flux du fluide de lavage sortant de l'orifice (11) vers la zone dudit ballonnet principal (4) et à favoriser le lavage de la trachée au niveau et à proximité dudit ballonnet principal (4), **caractérisé en ce que** :
- ledit ballonnet auxiliaire (13) est placé en position distale par rapport audit ballonnet principal (4).

2. Dispositif selon une ou plusieurs des revendications précédentes, en particulier la 1, dans lequel ledit ballonnet auxiliaire (13) a une longueur en direction axiale dudit tube (2) inférieure à la longueur dudit ballonnet principal (4).

3. Dispositif selon une ou plusieurs des revendications précédentes, en particulier la 1, dans lequel lesdits moyens de retenue auxiliaires (12) comprennent un deuxième lumen de gonflage (14) conçu pour gonfler et dégonfler ledit ballonnet auxiliaire (13) indépendamment dudit ballonnet principal (4).

4. Dispositif selon une ou plusieurs des revendications précédentes, en particulier la 1, dans lequel ledit lumen de lavage (9) comprend au moins un orifice (11) situé à proximité dudit ballonnet principal (4).

5. Dispositif selon une ou plusieurs des revendications précédentes, en particulier la 4, dans lequel ledit orifice (11) est situé en position distale par rapport audit ballonnet principal (4).

6. Dispositif selon une ou plusieurs des revendications précédentes, en particulier la 1, dans lequel ledit lumen de lavage (9) est partiellement placé à l'intérieur de la paroi (2b) dudit tube de ventilation (2).

7. Dispositif selon une ou plusieurs des revendications précédentes, en particulier la 1, comprenant un lumen distal (16) conçu pour permettre l'inoculation de substances directement à l'intérieur du système respiratoire du patient.
